# EUROPÄISCHE PATENTSCHRIFT

(11) **EP 0 577 024 B1**
(45) Veröffentlichungstag und Bekanntmachung des Hinweises auf die Patenterteilung: **16.10.1996**
(21) Anmeldenummer: 93110195.0
(22) Anmeldetag: 25.06.1993
(51) Int. Cl.: C07C 317/32, A61K 31/435, A61K 31/33, A61K 31/44

(54) **3,4,5-Substituierte Benzoylguanidine, Verfahren zu ihrer Herstellung, ihre Verwendung als Medikament oder Diagnostikum sowie sie enthaltendes Medikament**
3,4,5 substituted benzoylguanidines, process for their preparation, their use as medicament or diagnostic agent, as well as a medicament containing them
Benzoylguanidines 3,4,5 substituées, procédé pour leur préparation, leur utilisation comme médicament ou agent diagnostique, ainsi que médicament les contenant

(30) Priorität: 01.07.1992 DE 4221594; 22.07.1992 DE 4224107; 28.12.1992 DE 4224319
(43) Veröffentlichungstag der Anmeldung: 05.01.1994
(73) Patentinhaber: HOECHST AKTIENGESELLSCHAFT, 65926 Frankfurt am Main (DE)
(72) Erfinder: Weichert, Andreas, Dr., D-6000 Frankfurt/Main 70 (DE); Lang, Hans-Jochen, D-6238 Hofheim am Taunus (DE); Scholz, Wolfgang, Dr., D-6236 Eschborn (DE); Albus, Udo, Dr., D-6364 Florstadt 4 (DE); Lang, Florian, Prof. Dr., D-7778 Markdorf (DE)

(56) Entgegenhaltungen:
- EP-A- 0 416 499
- EP-A- 0 416 499
- EP-A- 0 556 674
- BIOCHEMICAL AND BIOPHYSICAL RESEARCH COMMUNICATIONS Bd. 184, Nr. 1, 15. April 1992, DULUTH, MINNESOTA US Seiten 112 - 117 ANDREAS SCHMID ET AL. 'Na+/H+ Exchange in porcine cerebral capillary endothelial cells is inhibited by a Benzoylguanidine derivative' *

## Beschreibung

3,4,5-Substituierte Benzoylguanidine, Verfahren zu ihrer Herstellung, ihre Verwendung als Medikament oder Diagnostikum sowie sie enthaltendes Medikament

Die Erfindung betrifft Benzoylguanidine der Formel I 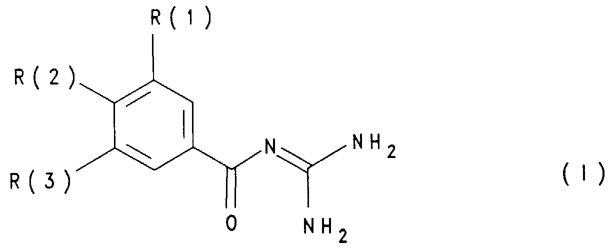 worin bedeuten:
R(1) R(4)-SOₘ oder R(5)R(6)N-SO₂-, mit
   m gleich Null, 1 oder 2,
   R(4) und R(5) gleich C₁-C₈-Alkyl, C₃-C₆-Alkenyl, -CₙH₂ₙ-R(7),
      n gleich Null, 1, 2, 3 oder 4,
      R(7) gleich C₅-C₇-Cycloalkyl, Phenyl, welches unsubstituiert ist oder substituiert mit 1-3 Substituenten aus der Gruppe
         F, Cl, CF₃, Methyl, Methoxy oder NR(8)R(9) mit R(8) und R(9) gleich H oder C₁-C₄-Alkyl,
      wobei R(5) auch in der Bedeutung von H steht,
   R(6) gleich H oder C₁-C₄-Alkyl,
      wobei R(5) und R(6) gemeinsam 4 oder 5 Methylengruppen sein können, von denen eine CH₂-Gruppe durch ein O, S, NH, N-CH₃ oder N-Benzyl ersetzt sein kann,
R(2) geradkettiges oder verzweigtes (C₅-C₈)-Alkyl, -CR(13) =CHR(12), -C≡CR(12) mit
   - R(12): gleich Phenyl, das unsubstituiert oder substituiert ist mit 1 - 3 Substituenten aus der Gruppe F, Cl, CF₃, Methyl, Methoxy oder NR(14)R(15) mit
   R(14) und R(15) gleich H oder (C₁-C₄)-Alkyl,
   oder
   - R(12): gleich (C₁-C₆)-Alkyl, das unsubstituiert oder mit 1-3 OH substituiert ist,
   oder
   - R(12): gleich (C₃-C₈)-Cycloalkyl;
   - R(13): gleich Wasserstoff oder Methyl,
oder
R(2) (C₃-C₈)-Cycloalkyl, (C₃-C₈)-Cycloalkyl-(C₁-C₄)-alkyl, Phenyl, C₆H₅-(C₁-C₄)-Alkyl, Naphthyl, Biphenylyl, 1,1-Diphenyl-(C₁-C₄)-Alkyl oder Cyclopentadienyl;
R(3) Wasserstoff, geradkettiges oder verzweigtes (C₅-C₈)-Alkyl, -CR(13) = CHR(12) oder -C≡CR(12) mit
   - R(12): gleich Phenyl, das unsubstituiert oder substituiert ist mit
   1 - 3 Substituenten aus der Gruppe F, Cl, CF₃, Methyl, Methoxy oder NR(14)R(15) mit R(14) und R(15) gleich H oder (C₁-C₄)-Alkyl,
   oder
   - R(12): gleich (C₁-C₆)-Alkyl; das unsubstituiert oder mit 1-3 OH substituiert ist,
   oder
   - R(12): gleich (C₃-C₈)-Cycloalkyl;
   - R(13): gleich Wasserstoff oder Methyl,
oder
- R(3): (C₃-C₈)-Cycloalkyl, (C₃-C₈)-Cycloalkyl-(C₁-C₄)-alkyl, Phenyl, C₆H₅-(C₁-C₄)-Alkyl, Naphthyl, Biphenylyl, 1,1-Diphenyl-(C₁-C₄)-Alkyl oder Cyclopentadienyl;
und wobei die Substituenten Phenyl, C₆H₅-(C₁-C₄)-Alkyl, Naphthyl, Biphenylyl und 1,1-Diphenyl-(C₁-C₄)-Alkyl in R(2) bzw. R(3) unsubstituiert oder mit 1-3 Substituenten aus den Gruppen F, Cl, CF₃, (C₁-C₄)-Alkyl, -Alkoxy, oder NR(10)R(11) mit R(10) und R(11) in der Bedeutung von H oder (C₁-C₄)-Alkyl substituiert sind,
sowie deren pharmazeutisch verträgliche Salze.

Bevorzugt sind Verbindungen I, in denen bedeuten:
R(1) R(4)-SOₘ oder R(5)R(6)N-SO₂-, mit m gleich Null, 1 oder 2,
   R(4) und R(5) gleich C₁-C₈-Alkyl, C₃-C₄-Alkenyl, -CₙH₂ₙ-R(7),
      n gleich Null oder 1,
         R(7) gleich C₅-C₆-Cycloalkyl, Phenyl, welches unsubstituiert ist oder substituiert mit 1-3 Substituenten aus der Gruppe
         F, Cl, CF₃, Methyl, Methoxy oder NR(8)R(9) mit R(8) und R(9) gleich H oder Methyl,
      wobei R(5) auch in der Bedeutung von H steht,
   R(6) gleich H oder Methyl,
      wobei R(5) und R(6) gemeinsam 4 oder 5 Methylengruppen sein können, von denen eine CH₂-Gruppe durch ein O, S, N-CH₃ oder N-Benzyl ersetzt sein kann,
R(2) geradkettiges oder verzweigtes (C₅-C₈)-Alkyl, (C₃-C₈)-Cycloalkyl, (C₃-C₈)-Cycloalkyl-(C₁ -C₂)-alkyl, Phenyl, C₆H₅-(C₁-C₂)-Alkyl, Naphthyl, Biphenylyl oder -C≡CR(12) mit
   - R(12): gleich Phenyl, das unsubstituiert oder substituiert ist mit 1 - 3 Substituenten aus der Gruppe F, Cl, CF₃, Methyl, Methoxy oder NR(14)R(15) mit
   R(14) und R(15) gleich H oder (C₁-C₄)-Alkyl;
   oder
   - R(12): gleich (C₁-C₆)-Alkyl;
   das unsubstituiert oder mit 1-3 OH substituiert ist,
   oder
   - R(12): gleich (C₃-C₈)-Cycloalkyl;
R(3) Wasserstoff, geradkettiges oder verzweigtes (C₅-C₈)-Alkyl, (C₃-C₈)-Cycloalkyl, (C₃-C₈)-Cycloalkyl-(C₁-C₂)-alkyl, Phenyl, C₆H₅-(C₁-C₂)-Alkyl, Naphthyl, Biphenylyl oder -C≡CR(12) mit
   - R(12): gleich Phenyl, das unsubstituiert oder substituiert ist mit 1 - 3 Substituenten aus der Gruppe F, Cl, CF₃, Methyl, Methoxy oder NR(14)R(15) mit
   R(14) und R(15) gleich H oder (C₁-C₄)-Alkyl;
   oder
   - R(12): gleich (C₁-C₆)-Alkyl,
   das unsubstituiert oder mit 1-3 OH substituiert ist,
   oder
   - R(12): gleich (C₃-C₈)-Cycloalkyl;
wobei die Substituenten Phenyl, C₆H₅-(C₁-C₂)-Alkyl, Naphthyl und Biphenylyl in R(2) bzw. R(3) unsubstituiert oder mit 1-3 Substituenten aus den Gruppen F, Cl, CF₃, (C₁-C₄)-Alkyl, -Alkoxy oder NR(10)R(11) mit R(10) und R(11) in der Bedeutung von H oder (C₁-C₄)-Alkyl substituiert sind,
sowie deren pharmazeutisch verträgliche Salze.

Besonders bevorzugt sind Verbindungen der Formel I, in denen bedeuten:
R(1) R(4)-SOₘ oder R(5)R(6)N-SO₂-, mit
   m gleich Null, 1 oder 2,
   R(4) gleich Methyl oder -CₙH₂ₙ-R(7),
      n gleich Null oder 1,
      R(7) gleich Phenyl, welches unsubstituiert ist oder substituiert mit 1-3 Substituenten aus der Gruppe
         Cl, CF₃, Methyl oder Methoxy,
   R(5) gleich H, C₁-C₆-Alkyl, Allyl, -CₙH₂ₙ-R(7),
      n gleich Null, 1, 2 oder 3,
      R(7) gleich Phenyl, welches unsubstituiert ist oder substituiert mit 1-3 Substituenten aus der Gruppe
         F, Cl, CF₃, Methyl, Methoxy oder NR(8)R(9) mit R(8) und R(9) gleich H oder Methyl,
   R(6) gleich H oder Methyl,
   wobei R(5) und R(6) gemeinsam 4 oder 5 Methylengruppen sein können, von denen eine CH₂-Gruppe durch ein O, S, N-CH₃ oder N-Benzyl ersetzt sein kann,
R(2) geradkettiges oder verzweigtes (C₅-C₈)-ACkyS, (C₃-C₈)-Cycloalkyl, (C₃-C₈)-Cycloalkyl-(C₁-C₂)-alkyl, Phenyl, C₆H₅-(C₁-C₂)-Alkyl, Naphthyl, Biphenylyl oder -C≡CR(12) mit
   - R(12): gleich Phenyl, das unsubstituiert oder substituiert ist mit 1 - 3 Substituenten aus der Gruppe F, Cl, CF₃, Methyl, Methoxy oder NR(14)R(15) mit
   R(14) und R(15) gleich H oder (C₁-C₄)-Alkyl,
   oder
   - R(12): gleich (C₁-C₆)-Alkyl;
   das unsubstituiert oder mit 1-3 OH substituiert ist,
   oder
   - R(12): gleich (C₃-C₈)-Cycloalkyl;
R(3) Wasserstoff, Phenyl, Cyclopentyl, C₆H₅-(C₁-C₂)-ASkyl, oder -C≡CR(12);
   - R(12): gleich Phenyl, das unsubstituiert oder substituiert ist mit
   1 - 3 Substituenten aus der Gruppe F, Cl, CF₃, Methyl, Methoxy oder NR(14)R(15) mit
   R(14) und R(15) gleich H oder (C₁-C₄)-Alkyl;
   oder
   - R(12): gleich (C₁-C₆)-Alkyl,
   das unsubstituiert oder mit 1-3 OH substituiert ist,
   oder
   - R(12): gleich (C₃-C₈)-Cycloalkyl;
wobei die Substituenten Phenyl, C₆H₅-(C₁-C₂)-Alkyl, Naphthyl und Biphenylyl in R(2) bzw. R(3) unsubstituiert oder mit 1-3 Substituenten aus den Gruppen F, Cl, CF₃, (C₁-C₄)-Alkyl, -Alkoxy, oder NR(10)R(11) mit R(10) und R(11) in der Bedeutung von H oder (C₁-C₄)-Alkyl substituiert sind,
sowie deren pharmazeutisch verträgliche Salze.

Enthält einer der Substituenten R(1) bis R(15) ein Asymmetriezentrum, so gehören sowohl S als auch R konfigurierte Verbindungen zur Erfindung. Die Verbindungen können als optische Isomere, als Diastereomere, als Racemate oder als Gemische derselben vorliegen.

Die Alkylreste können, soweit nicht anders erwähnt, sowohl geradkettig wie verzweigt vorliegen.

Die Erfindung betrifft weiterhin ein Verfahren zur Herstellung der Verbindungen I, dadurch gekennzeichnet, daß man

Verbindungen der Formel II 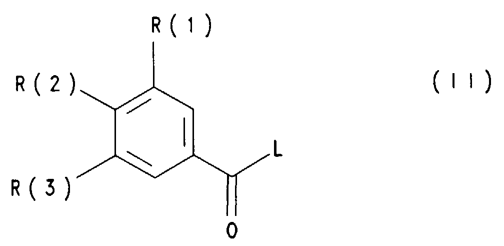 mit Guanidin umsetzt, worin R(1) bis R(3) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L=Cl), die man ihrerseits wiederum in an sich bekannter Welse aus den zugrundeliegenden Carbonsäuren (Formel II, L= OH) beispielsweise mit Thionylchlorid herstellen kann. Neben den Carbonsäurechloriden der Formel II (L=Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L=OCH₃ durch Behandeln mit gasförmigem HCI in Methanol, die Imidazolide der Formel II durch Behandeln mit Carbonyldiimidazol (L= 1-Imidazolyl, Staab, Angew. Chem. Int. Ed. Engl. 1, 351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyl uronium-tetrafluorborat ("TOTU")(Weiss und Krommer, Chemiker Zeitung 98, 817 (1974)). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L=OMe) mit Guanidin Methanol oder THF zwischen 20°C und Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II und Guanidin verwendet werden.

Wenn L = Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden, indem man beispielsweise 4-(bzw. 5-)Halogen-3-chlor-sulfonylbenzoesäuren mit Ammoniak oder Aminen in 3-Aminosulfonyl-4-(bzw. 5-)halogen-benzoesäuren bzw. mit einem schwachen Reduktionsmittel wie Natriumbisulfit und anschließender Alkylierung in 3-Alkylsulfonyl-4-(bzw. 5-)halogen-benzoesäuren überführt und die erhaltenen Benzoesäuren nach einer der oben beschriebenen Verfahrensvarianten zu erfindungsgemäßen Verbindungen I umgesetzt werden.

Die Einführung der Substituenten in 4- und 5-Stellung gelingt durch literaturbekannte Methoden des Palladium-vermittelten cross-couplings von Arylhalogeniden mit beispielsweise Organozink- bzw.
Organokupferverbindungen, Organostannanen, Organoboronsäuren oder Organoboranen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 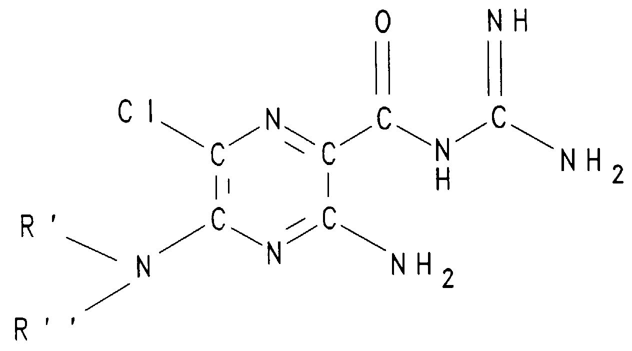 Amilorid: R',R"= H
Dimethylamilorid: R',R" = CH₃
Ethylisopropylamilorid: R' = C₂H₅, R" = CH(CH₃)₂

Darüberhinaus sind Untersuchungen bekannt geworden, die auf antiarrhythmische Eigenschaften von Amilorid hinweisen (Circulation 79, 1257-63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [(Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288), welche D 1 entspricht, sind

Benzoylguanidine beschrieben, die in der dem Rest R(3) entsprechenden Stellung ein Wasserstoffatom tragen. In der deutschen Patentanmeldung P 42 04 575.4 (HOE 92/F 034) werden 3,5-substituierte Benzoylguanidine vorgeschlagen, in welchen aber die Substituenten R(2) und R(3) nicht die nach der vorliegenden Erfindung beanspruchten Bedeutungen haben. Biochemical and Biophysical Research Communications 1992, 184, N. 1, Seite 112 - 117 beschreibt die Wirkung einer speziellen Verbindung nach US 5 091 394, nämlich der Verbindung 3-Methylsulfonyl-4-piperidino-benzoylguanidin (HOE 694) als Inhibitor des Na⁺/H⁺-Austauschs. Diese Verbindung zeigte bereits einen IC₅₀-Wert zwischen 1 und 10 x 10⁻⁶ Mol, sie ist also in der Wirksamkeit noch nicht befriedigend; vor allem hatte sie wegen des Piperidino-Ringes aber einige nicht akzeptable Nebenwirkungen.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindung auch als Arzneimittel zur Behandlung von Ischämien des Nervensystems, insbesondere des Zentral-Nervensystems, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindung der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers. Im Durchschnitt beträgt die tägliche Dosis einer Verbindung der Formel I bei einem etwa 75 kg schweren Patienten mindestens 0,001 mg/kg, vorzugsweise 0,01 mg/kg bis 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

Allgemeine Vorschrift zur Herstellung von Benzoyl-guanidinen (I) aus Benzoesäuren (II, L=OH)

0,01 M des Benzoesäurederivates der Formel II löst bzw. suspendiert man in 60 ml wasserfreiem Tetrahydrofuran (THF) und versetzt sodann mit 1,78 g (0,011 M) Carbonyldiimidazol. Nach dem Rühren über 2 Stunden bei Zimmertemperatur werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6-7 und filtriert das entsprechende Benzoylguanidin (Formel I) ab. Die so erhaltenen Benzoylguanidine können durch Behandeln mit wäßriger oder methanolischer Salzsäure oder anderen pharmakologisch verträglichen Säuren in die entsprechenden Salze übergeführt werden.

### Beispiel 1:

3-Methylsulfonyl-4-phenyl-benzoyl-guanidin-hydrochlorid: Farblose Kristalle, Schmp. 196-98°C

### Syntheseweg:

a) Reduktion von 4-Bromo-3-chlorsulfonyl-benzoesäure zu 2-Bromo-5-carboxy-benzolsulfinsäure mit Natriumdisulfit in Wasser bei 10-15°C und konstantem pH (8-9), acidifizieren mit HCI und abfiltrieren des Niederschlags, Schmp. 220-22°C
b) 2-Bromo-5-carboxy-benzolsulfinsäure-dinatriumsalz aus a) mit 2 Äquivalenten NaOH in Wasser/Methanol, eindampfen, suspendieren in Aceton und Kristalle abfiltrieren, Schmp. > 300°C
c) 4-Bromo-3-methylsulfonyl-benzoesäuremethylester aus b) mit 3,5 Äquivalenten Methyliodid in DMF bei 80°C/6 h umsetzen, Lösungsmittel abdestillieren, Rückstand in Wasser suspendieren, Kristalle absaugen, Smp. 135-37°C
d) 3-Methylsulfonyl-4-phenyl-benzoesäure aus c) mit 1.1 Äquivalenten Phenylboronsäure in wäßrigem Methanol/Toluol-Gemisch (Rückfluß, 3 h) in Gegenwart von katal. Palladiumacetat, Triphenylphosphin und Natriumcarbonat, Lösungsmittel abdestillieren, in Essigester aufnehmen, mit verd. Salzsäure neutral stellen, nach wäßriger Aufarbeitung Säulenchromatographie an Kieselgel mit Essigester/Cyclohexan (3:7).
e) 3-Methylsulfonyl-4-phenyl-benzoyl-guanidin-hydrochlorid aus d) nach allg. Vorschrift I (siehe oben). Farblose Kristalle, Smp. 196-98°C.

### Beispiel 2:

3-Methylsulfonyl-4-(2-naphthyl)-benzoyl-guanidin-hydrochlorid: Farbloses Pulver, amorph.

### Syntheseweg:

a) 3-Methylsulfonyl-4-(2-naphthyl)-benzoesäuremethylester aus 4-Bromo-3-methylsulfonyl-benzoesäuremethylester durch cross-coupling mit 2-Naphthylboronsäure analog Beispiel 1 d), farbloses Pulver, amorph.
b) 3-Methylsulfonyl-4-(2-naphthyl)-benzoyl-guanidin-hydrochlorid aus 2 a) durch Erhitzen zum Sieden in THF in Gegenwart von Guanidin und anschließende Hydrochloridbildung, farbloses Pulver, amorph.

### Beispiel 3:

3-Methylsulfonyl-4-(3-biphenyl)-benzoyl-guanidin-hydrochlorid: Sandfarbene Kristalle, Smp. 150-54°C.

### Syntheseweg:

a) 3-Methylsulfonyl-4-(3-biphenyl)benzoesäuremethylester aus 4-Bromo-3-methylsulfonylbenzoesäure-methylester durch cross-coupling mit 3-Biphenylboronsäure analog 1 d), leicht gelbliche Kristalle, Smp. 77-80°C.
b) 3-Methylsulfonyl-4-(3-biphenyl)-benzoyl-guanidin-hydrochlorid aus 3 a) durch Erhitzen zum Sieden in THF in Gegenwart von Guanidin und anschließende Hydrochloridbildung.

### Beispiel 4:

4-Cyclopropyl-3-methylsulfonyl-benzoylguanidin-hydrochlorid: Farblose Kristalle, Smp. 233-35°C.

### Syntheseweg:

a) 4-Cyclopropyl-3-methylsulfonyl-benzoesäuremethylester aus 4-Bromo-3-methylsulfonyl-benzoesäuremethylester (1 c) mit 1,5 Äquivalenten Cyclopropyl-tri-n-butylstannan in Hexamethylphosphorsäuretriamid (65°C, 6h) in Gegenwart von katal. Palladium(II)[1,1'-bis-(diphenylphosphino)-ferrocen]chlorid und Kupfer(I)iodid, wäßrige Aufarbeitung, Extraktion mit Essigester und anschließende Säulenchromatographie an Kieselgel mit Essigester/Cyclohexan (3:7), Smp. 81-83°C.
b) 4-Cyclopropyl-3-methylsulfonyl-benzoylguanidin-hydrochlorid aus 4 a) analog Beispiel 2 b), Smp. 233-35°C.

### Beispiel 5:

4-Cyclohexyl-3-methylsulfonyl-benzoylguanidin-hydrochlorid: Farblose Kristalle, Smp. 230-32°C.

### Syntheseweg:

a) 4-Cyclohexyl-3-methylsulfonyl-benzoesäuremethylester analog Beispiel 1 a-c) ausgehend von 4-Cyclohexyl-3-chlorsulfonyl-benzoesäure, Smp. 91-93°C.
b) 4-Cyclopropyl-3-methylsulfonyl-benzoylguanidin-hydrochlorid analog Beispiel 2 b), Smp. 230-32°C.

### Beispiel 7:

3-Methylsulfonyl-4-[(2'-phenyl)-ethinyl]-benzoylguanidin-hydrochlorid: Farblose Kristalle, Smp. 287°C.

### Syntheseweg:

a) 3-Methylsulfonyl-4-[(2'-phenyl)-ethinyl]-benzoesäuremethylester aus 4-Bromo-3-methylsulfonyl-benzoesäure-methylester (1 c) durch Stephans-Castro-coupling mit 2,5 Äquivalenten Phenylacetylen, Rühren bei Zimmertemperatur für 24 h in Gegenwart von katal. (5 mol.-%) Bis(Triphenylphosphin)-palladium(11)chlorid,15 mol.-% Kupfer(I)iodid und 3 Äquivalenten n-Butylamin, wäßrige Ammoniumchloridaufarbeitung, Extraktion mit Essigester und anschließende Säulenchromatographie an Kieselgel mit Essigester/Cyclohexan (3:7), farblose Kristalle, Smp. 138-39°C.
b) 3-Methylsulfonyl-4-[(2'-phenyl)-ethinyl]-benzoylguanidin-hycrochlorid aus 7 a) analog Beispiel 2 b).

### Beispiel 8:

4-[(2'-Cyclohexyl)-ethinyl]-3-methylsulfonyl-benzoylguanidin-hydrochlorid: Farblose Kristalle, Smp. 224-25°C.

### Syntheseweg:

a) 4-[(2'-Cyclohexyl)-ethinyl]-3-methylsulfonyl-benzoesäuremethylester aus 4-Bromo-3-methylsulfonyl-benzoesäure-methylester (1 c) durch Stephans-Castro-coupling wie für 7 a) beschrieben, Kopplungspartner Cyclohexylacetylen, farblose Kristalle, Smp. 81-82°C.
b) 4-[(2'-Cyclohexyl)-ethinyl]-3-methylsulfonyl-benzoylguanidin-hydrochlorid aus 8 a) analog Beispiel 2 b).

### Beispiel 9:

3-Methylsulfonyl-4-phenylethyl-benzoylguanidin-hydrochlorid: Farblose Kristalle, Smp. 230-31°C.

### Syntheseweg:

a) 3-Methylsulfonyl-4-phenylethyl-benzoesäuremethylester aus 7 a) durch Palladium/Kohle-Hydrierung unter Normaldruck in Methanol innerhalb 1 h, farblose Kristalle, Smp. 91-93°C.
b) 3-Methylsulfonyl-4-phenylethyl-benzoylguanidin-hydrochlorid aus 9 a) analog Beispiel 2 b).

### Beispiel 10:

4-Cyclopentyl-3-methylsulfonyl-benzoylguanidin-hydrochlorid: Farblose Kristalle, Smp. 243-45°C.

### Syntheseweg:

a) 4-Cyclopentyl-3-methylsulfonyl-benzoesäuremethylester aus 4-Bromo-3-methylsulfonyl-benzoesäuremethylester (1 c) durch cross-coupling mit 1,5 Äquivalenten Cyclopentylzinkchlorid (aus Cyclopentylmagnesiumchlorid durch Transmetallierung mit Zink(II)chlorid-Etherat in THF) durch Rühren bei Zimmertemperatur in Gegenwart von katal. Palladium(II)[1,1'-bis-(diphenylphosphino)-ferrocen]chlorid und Kupfer(I)iodid, wäßrige Aufarbeitung, Extraktion mit Essigester und anschließende Säulenchromatographie an Kieselgel mit Essigester/n-Heptan (3:7), farblose Kristalle, Smp. 143-44°C.
b) 4-Cyclopentyl-3-methylsulfonyl-benzoylguanidin-hydrochlorid aus 10 a) analog Beispiel 2 b).

### Beispiel 11:

4-Benzyl-3-methylsulfonyl-benzoylguanidin-hydrochlorid : Farblose Kristalle, Smp. 284°C

### Syntheseweg:

a) 4-Benzyl-3-methylsulfonyl-benzoesäuremethylester aus 4-Bromo-3-methylsulfonyl-benzoesäure-methylester (1 c) durch cross-coupling mit 1,5 Äquivalenten Benzylzinkchlorid (aus Benzyl-magnesiumchlorid durch Transmetallierung mit Zink(ll)chlorid-Etherat in THF), Erhitzen zum Sieden für 2 h in Gegenwart von katal. (5 mol.-%) Palladium(II)acetat und Triphenylphosphin, wäßrige Aufarbeitung, Extraktion mit Essigester und anschließende Säulenchromatographie an Kieselgel mit Essigester/Cyclohexan (3:7), farblose Kristalle, Smp. 104-106°C.
b) 4-Benzyl-3-methylsulfonyl-benzoylguanidin-hydrochlorid aus 11 a) analog Beispiel 2 b).

### Pharmakologische Daten:

Inhibitoren des Na⁺/H⁺-Exchangers von Kaninchenerythrozyten:

Weiße Neuseeland-Kaninchen (Ivanovas) erhielten eine Standard-Diät mit 2 % Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l : 140 NaCl, 3 KCl, 150 Sucrose, 0,1 Ouabain, 20 Tris-hydroxymethylaminomethan) bei pH 7,4 und 37°C inkubiert. Die Erythrozyten wurden danach dreimal mit eiskalter MgCl₂-Ouabain-Lösung (mmol/l : 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrozyten nach Inkubation errechnet. Der Amilorid-hemmbare Natrium-lnflux ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse
Inhibition des Na⁺/H⁺-Exchangers:

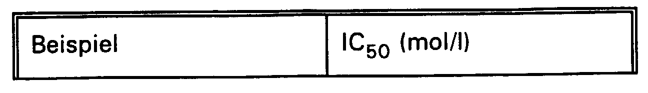

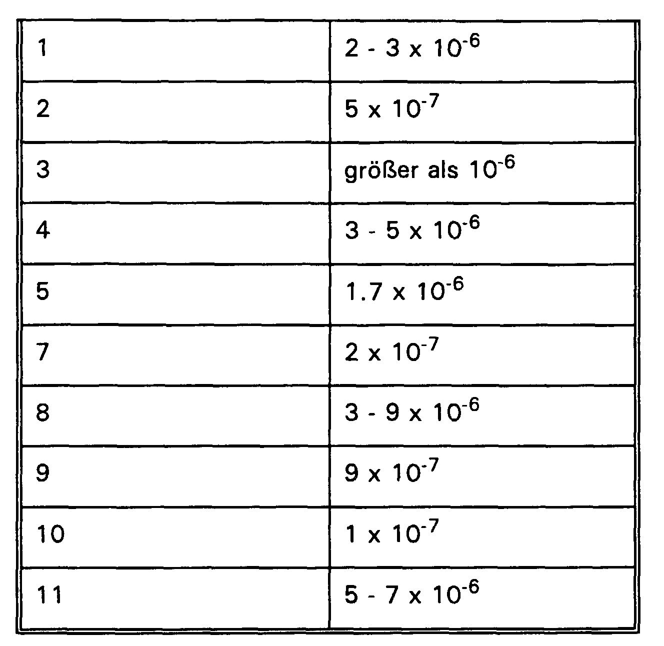

## Patentansprüche

1. Benzoylguanidine der Formel I 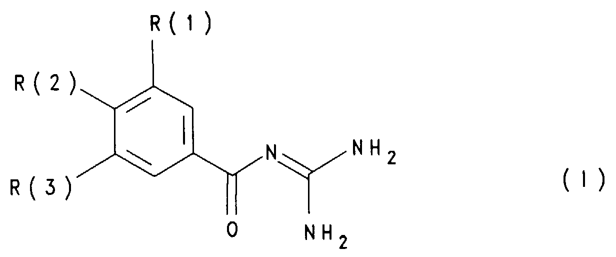 worin bedeuten:
R(1) R(4)-SOₘ oder R(5)R(6)N-SO₂-;
m Null, 1 oder 2;
R(4) und R(5)
C₁-C₈-Alkyl, C₃-C₆-Alkenyl oder -CₙH₂ₙBR(7);
n Null, 1, 2, 3 oder 4;
R(7) C₅-C₇-Cycloalkyl oder Phenyl, welches unsubstituiert ist oder substituiert mit 1 - 3 Substituenten aus der Gruppe F, Cl, CF₃, Methyl, Methoxy und NR(8)R(9);
R(8) und R(9)
H oder C₁-C₄-Alkyl;
oder
R(5) H;
R(6) H oder C₁-C₄-Alkyl;
oder
R(5) und R(6)
gemeinsam 4 oder 5 Methylengruppen, von denen eine CH₂-Gruppe durch ein O, S, NH, N-CH₃ oder N-Benzyl ersetzt sein kann,
R(2) geradkettiges oder verzweigtes (C₅-C₈)-Alkyl, -CR(13)=CHR(12) oder -C≡CR(12);
R(12) Phenyl,
das unsubstituiert oder substituiert ist mit 1 - 3 Substituenten aus der Gruppe F, Cl, CF₃, Methyl, Methoxy oder NR(14)R(15);
R(14) und R(15)
H oder (C₁-C₄)-Alkyl; oder
R(12) (C₁-C₆)-Alkyl,
das unsubstituiert oder mit 1 - 3 OH substituiert ist, oder
R(12) (C₃-C₈)-Cycloalkyl;
R(13) Wasserstoff oder Methyl,
oder
R(2) (C₃-C₈)-Cycloalkyl, (C₃-C₈)-Cycloalkyl-(C₁-C₄)-alkyl, Phenyl, C₆H₅-(C₁-C₄)-Alkyl, Naphthyl, Biphenylyl, 1,1-Diphenyl-(C₁-C₄)-Alkyl oder Cyclopentadienyl;
R(3) Wasserstoff, geradkettiges oder verzweigtes (C₅-C₈)-Alkyl, -CR(13) = CHR(12) oder -C≡CR(12);
R(12) Phenyl,
das unsubstituiert oder substituiert ist mit 1 - 3 Substituenten aus der Gruppe F, Cl, CF₃, Methyl, Methoxy oder NR(14)R(15);
R(14) und R(15)
H oder (C₁-C₄)-Alkyl;
oder
R(12) (C₁-C₆)-Alkyl,
das unsubstituiert oder mit 1-3 OH substituiert ist;
oder
R(12) (C₃-C₈)-Cycloalkyl;
R(13) Wasserstoff oder Methyl;
oder
R(3) (C₃-C₈)-Cycloalkyl, (C₃-C₈)-Cycloalkyl-(C₁-C₄)-alkyl, Phenyl, C₆H₅-(C₁-C₄)-Alkyl, Naphthyl, Biphenylyl, 1,1-Diphenyl-(C₁-C₄)-Alkyl oder Cyclopentadienyl;
und wobei die Substituenten Phenyl, C₆H₅-(C₁-C₄)-Alkyl, Naphthyl, Biphenylyl und 1,1-Diphenyl-(C₁-C₄)-Alkyl in R(2) bzw. R(3) unsubstituiert oder mit 1-3 Substituenten aus den Gruppen F, Cl, CF₃, (C₁-C₄)-Alkyl, -Alkoxy, oder NR(10)R(11) mit R(10) und R(11) in der Bedeutung von H oder (C₁-C₄)-Alkyl substituiert sind,
sowie deren pharmazeutisch verträgliche Salze.

2. Verbindung nach Anspruch 1, dadurch gekennzeichnet, daß bedeuten:
R(1) R(4)-SOₘ oder R(5)R(6)N-SO₂-;
m Null, 1 oder 2;
R(4) und R(5)
C₁-C₈-Alkyl, C₃-C₄-Alkenyl, -CₙH₂ₙ-R(7);
n Null oder 1;
R(7) C₅-C₆-Cycloalkyl, Phenyl,
welches unsubstituiert ist oder substituiert mit 1 - 3 Substituenten aus der Gruppe F, Cl, CF₃, Methyl, Methoxy oder NR(8)R(9);
R(8) und R(9)
H oder Methyl;
oder
R(5) H;
R(6) H oder Methyl;
oder
R(5) und R(6)
gemeinsam 4 oder 5 Methylengruppen, von denen eine CH₂-Gruppe durch ein O, S, N-CH₃ oder N-Benzyl ersetzt sein kann,
R(2) geradkettiges oder verzweigtes (C₅-C₈)-Alkyl, (C₃-C₈)-Cycloalkyl, (C₃-C₈)-Cycloalkyl-(C₁-C₂)-alkyl, Phenyl, C₆H₅-(C₁-C₂)-Alkyl, Naphthyl, Biphenylyl oder -C≡CR(12);
R(12) Phenyl,
das unsubstituiert oder substituiert ist mit 1 - 3 Substituenten aus der Gruppe F, Cl, CF₃, Methyl, Methoxy und NR(14)R(15);
R(14) und R(15)
H oder (C₁-C₄)-Alkyl; oder
R(12) (C₁-C₆)-Alkyl,
das unsubstituiert oder mit 1 - 3 OH substituiert ist;
oder
R(12) (C₃-C₈)-Cycloalkyl;
R(3) Wasserstoff, geradkettiges oder verzweigtes (C₅-C₈)-Alkyl, (C₃-C₈)-Cycloalkyl, (C₃-C₈)-Cycloalkyl-(C₁-C₂)-alkyl, Phenyl, C₆H₅-(C₁-C₂)-Alkyl, Naphthyl, Biphenylyl oder -C≡CR(12);
R(12) Phenyl,
das unsubstituiert oder substituiert ist mit 1 - 3 Substituenten aus der Gruppe F, Cl, CF₃, Methyl, Methoxy und NR(14)R(15);
R(14) und R(15)
H oder (C₁-C₄)-Alkyl;
oder
R(12) (C₁-C₆)-Alkyl,
das unsubstituiert oder mit 1 - 3 OH substituiert ist;
oder
R(12) (C₃-C₈)-Cycloalkyl;
wobei die Substituenten Phenyl, C₆H₅-(C₁-C₂)-Alkyl, Naphthyl und Biphenylyl in R(2) bzw. R(3) unsubstituiert oder mit 1 - 3 Substituenten aus den Gruppen F, Cl, CF₃, (C₁-C₄)-Alkyl, -Alkoxy oder NR(10)R(11) mit R(10) und R(11) in der Bedeutung von H oder (C₁-C₄)-Alkyl substituiert sind,
sowie deren pharmazeutisch verträgliche Salze.

3. Verbindung nach Anspruch 1, dadurch gekennzeichnet, daß bedeuten:
R(1) R(4)-SOₘ oder R(5)R(6)N-SO₂-;
m Null, 1 oder 2;
R(4) Methyl oder-CₙH₂ₙ-R(7);
n Null oder 1;
R(7) Phenyl,
welches unsubstituiert ist oder substituiert mit 1 - 3 Substituenten aus der Gruppe Cl, CF₃, Methyl und Methoxy;
R(5) H, C₁-C₆-Alkyl, Allyl oder -CₙH₂ₙ-R(7);
n Null, 1, 2 oder 3;
R(7) Phenyl,
welches unsubstituiert ist oder substituiert mit 1 - 3 Substituenten aus der Gruppe F, Cl, CF₃, Methyl, Methoxy oder NR(8)R(9); R(8) und R(9)
H oder Methyl;
R(6) H oder Methyl;
oder
R(5) und R(6)
gemeinsam 4 oder 5 Methylengruppen, von denen eine CH₂-Gruppe durch ein O, S, N-CH₃ oder N-Benzyl ersetzt sein kann,
R(2) geradkettiges oder verzweigtes (C₅-C₈)-ACkyS, (C₃-C₈)-Cycloalkyl, (C₃-C₈)-Cycloalkyl-(C₁-C₂)-alkyl, Phenyl, C₆H₅-(C₁-C₂)-Alkyl, Naphthyl, Biphenylyl oder -C≡CR(12);
R(12) Phenyl,
das unsubstituiert oder substituiert ist mit 1 - 3 Substituenten aus der Gruppe F, Cl, CF₃, Methyl, Methoxy und NR(14)R(15);
R(14) und R(15)
H oder (C₁-C₄)-Alkyl;
oder
R(12) (C₁-C₆)-Alkyl,
das unsubstituiert oder mit 1 - 3 OH substituiert ist;
oder
R(12) (C₃-C₈)-Cycloalkyl;
R(3) Wasserstoff, Phenyl, Cyclopentyl, C₆H₅-(C₁-C₂)-Alkyl, oder -C≡CR(12);
R(12) Phenyl,
das unsubstituiert oder substituiert ist mit 1 - 3 Substituenten aus der Gruppe F, Cl, CF₃, Methyl, Methoxy und NR(14)R(15);
R(14) und R(15)
H oder (C₁-C₄)-Alkyl;
oder
R(1 2) (C₁-C₆)-Alkyl,
das unsubstituiert oder mit 1 - 3 OH substituiert ist;
oder
R(12) (C₃-C₈)-Cycloalkyl;
wobei die Substituenten Phenyl, C₆H₅-(C₁-C₂)-Alkyl, Naphthyl und Biphenylyl in R(2) bzw. R(3) unsubstituiert oder mit 1-3 Substituenten aus den Gruppen F, Cl, CF₃, (C₁-C₄)-Alkyl, -Alkoxy, oder NR(10)R(11) mit R(10) und R(11) in der Bedeutung von H oder (C₁-C₄)-Alkyl substituiert sind,
sowie deren pharmazeutisch verträgliche Salze.

4. Verfahren zur Herstellung der Verbindungen I, dadurch gekennzeichnet, daß man
Verbindungen der Formel II 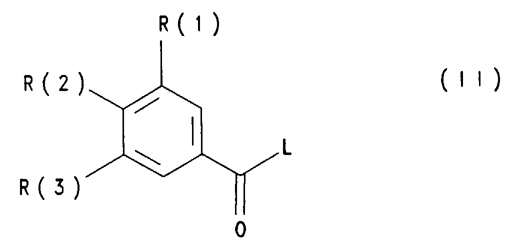 mit Guanidin umsetzt, worin R(1) bis R(3) die angegebene Bedeutung besitzen und L für eine leicht nucleophil substituierbare leaving group steht.

5. Verwendung einer Verbindung I nach Anspruch 1 zur Herstellung eines Medikaments zur Behandlung von Arrhythmien.

6. Verwendung einer Verbindung I nach Anspruch 1 zur Herstellung eines Medikaments zur Behandlung oder Prophylaxe des Herzinfarkts.

7. Verwendung einer Verbindung I nach Anspruch 1 zur Herstellung eines Medikaments zur Behandlung der angina pectoris.

8. Verwendung einer Verbindung I nach Anspruch I zur Herstellung eines Medikaments zur Behandlung von ischämischen Zuständen des Herzens.

9. Verwendung einer Verbindung I nach Anspruch I zur Herstellung eines Medikaments zur Behandlung von Krankheiten, bei denen die Proliferation der Fibroblasten eine primäre oder sekundäre Ursache darstellt, und somit ihre Verwendung als Antiatherosklerotika, Mittel gegen diabetische Spätkomplikationen, Krebserkrankungen, fibrotische Erkrankungen wie Lungenfibrose, Leberfibrose oder Nierenfibrose.

10. Verwendung einer Verbindung I nach Anspruch 1 zur Herstellung eines wissenschaftliches Tools zur Inhibition des Na⁺/H⁺ Exchangers zur Diagnose der Hypertonie und proliferativer Erkrankungen.

## Claims

1. A benzoylguanidine of the formula I 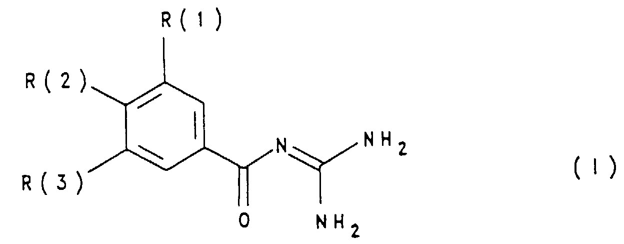 in which:
R(1) is R(4)-SOₘ or R(5)R(6)N-SO₂-;
m is zero, 1 or 2;
R(4) and R(5) are C₁-C₈-aSky-, C₃-C₆-alkenyl or -CₙH₂ₘ-R(7);
n is zero, 1, 2, 3 or 4;
R(7) is C₅-C₇-cycloalkyl, or phenyl which is unsubstituted or substituted by 1-3 substituents from the group consisting of F, Cl, CF₃, methyl, methoxy and NR(8)R(9);
R(8) and R(9) are H or C₁-C₄-alkyl;
or
R(5) is H;
R(6) is H or C₁-C₄-alkyl;
or
R(5) and R(6) together are 4 or 5 methylene groups, of which one CH₂ group can be replaced by an O, S, NH, N-CH₃ or N-benzyl,
R(2) is straight-chain or branched (C₅-C₈)-alkyl, -CR(13)=CHR(12) or -C=CR(12);
R(12) is phenyl which is unsubstituted or substituted by 1-3 substituents from the group consisting of F, Cl, CF₃, methyl, methoxy and NR(14)R(15);
R(14) and R(15) are H or (C₁-C₄)-alkyl,
or
R(12) is (C₁-C₆)-alkyl;
which is unsubstituted or substituted by 1-3 OH,
or
R(12) is (C₃-C₈)-cycloalkyl;
R(13) is hydrogen or methyl,
or
R(2) is (C₃-C₈)-cycloalkyl, (C₃-C₈)-cycloalkyl-(C₁-C₄)-alkyl, phenyl, C₆H₅-(C₁-C₄)-alkyl, naphthyl, biphenylyl, 1,1-diphenyl-(C₁-C₄)-alkyl or cyclopentadienyl;
R(3) is straight-chain or branched (C₅-C₈)-alkyl, -CR(13)=CHR(12) or -C≡CR(12);
R(12) is phenyl which is unsubstituted or substituted by 1-3 substituents from the group consisting of F, Cl, CF₃, methyl, methoxy and NR(14)R(15);
R(14) and R(15) are H or (C₁-C₄)-alkyl,
or
R(12) is (C₁-C₆)-alkyl;
which is unsubstituted or substituted by 1-3 OH,
or
R(12) is (C₃-C₈)-cycloalkyl;
R(13) is hydrogen or methyl
or
R(3) is (C₃-C₈)-cycloalkyl, (C₃-C₈)-cycloalkyl-(C₁-C₄)-alkyl, phenyl, C₆H₅-(C₁-C₄)-alkyl, naphthyl, biphenylyl, 1,1-diphenyl-(C₁-C₄)-alkyl or cyclopentadienyl;
and where the substituents phenyl, C₆H₅(-C₁-C₄)-alkyl, naphthyl, biphenylyl and 1,1-diphenyl-(C₁-C₄)-alkyl in R(2) and R(3) are unsubstituted or substituted by 1-3 substituents from the group consisting of F, Cl, CF₃, (C₁-C₄)-alkyl or -alkoxy, and NR(10)R(11) where R(10) and R(11) are H or (C₁-C₄)-alkyl,
or its pharmaceutically tolerable salts.

2. A compound as claimed in claim 1, wherein:
R(1) is R(4)-SOₘ or R(5)R(6)N-SO₂-;
m is zero, 1 or 2;
R(4) and R(5) are C₁-C₈-alkyl, C₃-C₄-alkenyl or -CₙH₂ₘ-R(7);
n is zero or 1;
R(7) is C₅-C₆-cycloalkyl, or phenyl which is unsubstituted or substituted by 1-3 substituents from the group consisting of F, Cl, CF₃, methyl, methoxy and NR(8)R(9);
R(8) and R(9) are H or methyl;
or
R(5) is H;
R(6) is H or methyl;
or
R(5) and R(6) together are 4 or 5 methylene groups, of which one CH₂ group can be replaced by an O, S, N-CH₃ or N-benzyl,
R(2) is straight-chain or branched (C₅-C₈)-alkyl, (C₃-C₈)-cycloalkyl, (C₃-C₈)-cycloalkyl-(C₁-C₂)-alkyl, phenyl, C₆H₅-(C₁-C₂)-alkyl, naphthyl, biphenylyl or -C≡CR(12);
R(12) is phenyl which is unsubstituted or substituted by 1-3 substituents from the group consisting of F, Cl, CF₃, methyl, methoxy and NR(14)R(15);
R(14) and R(15) are H or (C₁-C₄)-alkyl;
or
R(12) is (C₁-C₆)-alkyl which is unsubstituted or
substituted by 1-3 OH;
or
R(12) is (C₃-C₈)-cycloalkyl;
R(3) is straight-chain or branched (C₅-C₈)-alkyl, (C₃-C₈)-cycloalkyl, (C₃-C₈)-cycloalkyl-(C₁-C₂)-alkyl, phenyl, C₆H₅-(C₁-C₂)-alkyl, naphthyl, biphenylyl or -C≡CR(12);
R(12) is phenyl which is unsubstituted or substituted by 1-3 substituents from the group consisting of F, Cl, CF₃, methyl, methoxy and NR(14)R(15);
R(14) and R(15) are H or (C₁-C₄)-alkyl;
or
R(12) is (C₁-C₆)-alkyl which is unsubstituted or
substituted by 1-3 OH; or
R(12) is (C₃-C₈)-cycloalkyl;,
where the substituents phenyl, C₆H₅-(C₁-C₂)-alkyl, naphthyl and biphenylyl in R(2) and R(3) are unsubstituted or substituted by 1-3 substituents from the group consisting of F, Cl, CF₃, (C₁-C₄)-alkyl or -alkoxy, and NR(10)R(11) where R(10) and R(11) are H or (C₁-C₄)-alkyl,
or its pharmaceutically tolerable salts.

3. A compound as claimed in claim 1, wherein:
R(1) is R(4)-SOₘ or R(5)R(6)N-SO₂-;
m is zero, 1 or 2;
R(4) is methyl or -CₙH₂ₘ-R(7);
n is zero or 1;
R(7) is phenyl which is unsubstituted or substituted by 1-3 substituents from the group consisting of Cl, CF₃, methyl and methoxy;
R(5) is H, C₁-C₆-alkyl, allyl or -CₙH₂ₙ-R(7);
n is zero, 1, 2 or 3;
R(7) is phenyl which is unsubstituted or substituted by 1-3 substituents from the group consisting of F, Cl, CF₃, methyl, methoxy and NR(8)R(9);
R(8) and R(9) are H or methyl;
R(6) is H or methyl,
or
R(5) and R(6) together are 4 or 5 methylene groups, of which one CH₂ group can be replaced by an O, S, N-CH₃ or N-benzyl,
R(2) is straight-chain or branched (C₅-C₈)-alkyl, (C₃-C₈)-cycloalkyl, (C₃-C₈)-cycloalkyl-(C₁-C₂)-alkyl, phenyl, C₆H₅-(C₁-C₂)-alkyl, naphthyl, biphenylyl or -C≡CR(12);
R(12) is phenyl which is unsubstituted or substituted by 1-3 substituents from the group consisting of F, Cl, CF₃, methyl, methoxy and NR(14)R(15);
R(14) and R(15) are H or (C₁-C₄)-alkyl,
or
R(12) is (C₁-C₆)-alkyl which is unsubstituted or substituted by 1-3 OH;
or
R(12) is (C₃-C₈)-cycloalkyl;
R(3) is hydrogen, phenyl, cyclopentyl, C₆H₅-(C₁-C₂)-alkyl, or -C≡CR(12);
R(12) is phenyl which is unsubstituted or substituted by 1-3 substituents from the group consisting of F, Cl, CF₃, methyl, methoxy and NR(14)R(15);
R(14) and R(15) are H or (C₁-C₄)-alkyl,
or
R(12) is (C₁-C₆B-alkyl which is unsubstituted or substituted by 1-3 OH,
or
R(12) is (C₃-C₈)-cycloalkyl;
where the substituents phenyl, C₆H₅-(C₁-C₂)-alkyl, naphthyl and biphenylyl in R(2) and R(3) are unsubstituted or substituted by 1-3 substituents from the group consisting of F, Cl, CF₃, (C₁-C₄)-alkyl or - alkoxy, and NR(10)R(11) where R(10) and R(11) are H or (C₁-C₄)-alkyl,
or its pharmaceutically tolerable salts.

4. A process for the preparation of a compound I, which comprises
reacting a compound of the formula II 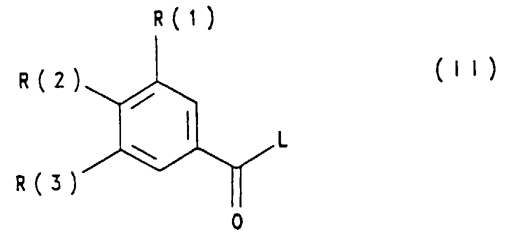 with guanidine, in which R(1) to R(3) have the given meaning and L is a leaving group which can be easily nucleophilically substituted.

5. The use of a compound I as claimed in claim 1 for the production of a medicament for the treatment of arrhythmias.

6. The use of a compound I as claimed in claim 1 for the production of a medicament for the treatment or prophylaxis of cardiac infarct.

7. The use of a compound I as claimed in claim 1 for the production of a medicament for the treatment of angina pectoris.

8. The use of a compound I as claimed in claim 1 for the production of a medicament for the treatment of ischemic conditions of the heart.

9. The use of a compound I as claimed in claim 1 for the production of a medicament for the treatment of diseases in which the proliferation of fibroblasts is a primary or secondary cause, and hence their use as antiatherosclerotics, agents against diabetic late complications, cancers, and fibrotic diseases such as pulmonary fibrosis, fibrosis of the liver or fibrosis of the kidneys.

10. The use of a compound I as claimed in claim 1 for the production of a scientific tool to inhibit the Na⁺/H⁺ exchanger to diagnose hypertension and proliferative diseases.

## Revendications

1. Benzoylguanidines de formule I 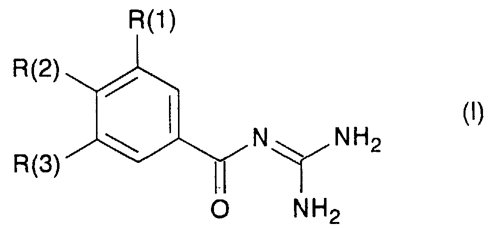 dans laquelle les groupes ont les significations suivantes :
R(1) : R(4)-SOₘ ou R(5)R(6)N-SO₂- ;
m: 0, 1 ou 2 ;
R(4) et R(5) :
groupe alkyle-(C₁-C₈), alcényle-(C₃-C₆) ou -CₙH₂ₙ-R(7) ,
n : 0, 1, 2, 3 ou 4 ;
R(7) : groupe cycloalkyle-(C₅-C₇) ou phényle, celui-ci étant non substitué ou substitué par 1 à 3 substituants choisis parmi F, CI et les groupes CF₃, méthyle, méthoxy et NR(8)R(9) ;
R(8) et R(9) :
H ou un groupe alkyle-(C₁-C₄) ;
ou bien
R(5) : H ;
R(6) : H ou un groupe alkyle-(C₁-C₄) ;
ou bien
R(5) et R(6)
pouvant représenter ensemble 4 ou 5 groupes méthylène parmi lesquels un groupe CH₂ peut être remplacé par O, S, NH, N-CH₃ ou un groupe N-benzyle,
R(2): groupe alkyle-(C₅-C₈) linéaire ou ramifié, -CR(13)=CHR(12) ou -C≡CR(12) ;
R(12): phényle
non substitué ou substitué par 1 à 3 substituants choisis parmi F, CI et les groupes CF₃, méthyle, méthoxy et NR(14)R(15) ;
R(14) et R(15) :
H ou un groupe alkyle-(C₁-C₄) ;
ou bien
R(12) : groupe alkyle-(C₁-C₆)
non substitué ou substitué par 1 à 3 groupes OH,
ou bien
R(12) : groupe cycloalkyle-(C₃-C₈) ;
R(13) : atome d'hydrogène ou groupe méthyle,
ou bien
R(2) : groupe cycloalkyle-(C₃-C₈), cycloalkyl-(C₃-C₈)-alkyle-(C₁-C₄), phényle, C₆H₅-alkyle-(C₁-C₄),naphtyle,biphénylyle,1,1-diphényl-alkyle-(C₁-C₄) ou cyclopentadiényle ;
R(3): atome d'hydrogène, un groupe alkyle-(C₅-C₈) linéaire ou ramifié, -CR(13)=CHR(12) ou -C≡CR(12),
R(12) : groupe phényle
non substitué ou substitué par 1 à 3 substituants choisis parmi F, CI et les groupes CF₃, méthyle, méthoxy et NR(14)R(15),
R(14) et R(15) :
H ou groupe alkyle-(C₁-C₄),
ou bien
R(12) : groupe alkyle-(C₁-C₆)
non substitué ou substitué par 1 à 3 groupes OH,
ou bien
R(12) : groupe cycloalkyle-(C₃-C₈) ;
R(13) : atome d'hydrogène ou groupe méthyle,
ou bien
R(3) : groupe cycloalkyle-(C₃-C₈), cycloalkyl-(C₃-C₈)-alkyle-(C₁-C₄), phényle, C₆H₅-alkyle-(C₁-C₄), naphtyle, biphénylyle, 1,1 diphényl-alkyle-(C₁-C₄) ou cyclopentadiényle;
et où les substituants phényle, C₆H₅-alkyle-(C₁-C₄), naphtyle, biphénylyle et 1,1 -diphényl-alkyle-(C₁-C₄), dans les groupes R(2) et R(3), ne sont pas substitués ou sont substitués par 1 à 3 substituants choisis parmi F, CI et les groupes CF₃, alkyle-(C₁-C₄), alcoxy-(C₁-C₄) et NR(10)R(11), et R(11) signifiant H ou un groupe alkyle-(C₁-C₄),
ainsi que leurs sels pharmaceutiquement acceptables.

2. Composé selon la revendication 1, caractérisé en ce que les groupes ont les significations suivantes :
R(1) : R(4)-SOₘ ou R(5)R(6)N-SO₂- ;
m : 0, 1 ou 2 ;
R(4) et R(5) :
groupe alkyle-(C₁-C₈), alcényle-(C₃-C₄), -CₙH₂ₙ-R(7) ;
n : 0 ou 1 ;
R(7) : groupe cycloalkyle-(C₅-C₆), phényle, celui-ci étant non substitué ou substitué par 1 à 3 substituants choisis parmi F, CI et les groupes CF₃, méthyle, méthoxy et NR(8)R(9) ;
R(8) et R(9) :
H ou groupe méthyle ;
ou bien
R(5) : H,
R(6) : H ou groupe méthyle,
ou bien
R(5) et R(6)
pouvant représenter ensemble 4 ou 5 groupes méthylène parmi lesquels un groupe CH₂ peut être remplacé par O, S, N-CH₃ ou un groupe N-benzyle,
R(2) : groupe alkyle-(C₅-C₈) linéaire ou ramifié, cycloalkyle-(C₃-C₈), cycloalkyl-(C₃-C₈)-alkyle-(C₁-C₂), phényle, C₆H₅-alkyle-(C₁-C₂), naphtyle, biphénylyle ou -C≡CR(12) ;
R(12) : groupe phényle
non substitué ou substitué par 1 à 3 substituants choisis parmi F, CI et les groupes CF₃, méthyle, méthoxy et NR(14)R(15) ;
R(14) et R(15) :
H ou groupe alkyle-(C₁-C₄) ; ou bien
R(12) : groupe alkyle-(C₁-C₆)
non substitué ou substitué par 1 à 3 groupes OH ;
ou bien
R(12) : groupe cycloalkyle-(C₃-C₈) ;
R(3): atome d'hydrogène, groupe alkyle-(C₅-C₈) linéaire ou ramifié, cycloalkyle-(C₃-C₈), cycloalkyl-(C₃-C₈)-alkyle-(C₁-C₂), phényle, C₆H₅-alkyle-(C₁-C₂), naphtyle, biphénylyle ou -C≡CR(12),
R(12) : groupe phényle
non substitué ou substitué par 1 à 3 substituants choisis parmi F, CI et les groupes CF₃, méthyle, méthoxy et NR(14)R(15) ;
R(14) et R(15) :
H ou groupe alkyle-(C₁-C₄) ;
ou bien
R(12) : groupe alkyle-(C₁-C₆)
non substitué ou substitué par 1 à 3 groupes OH,
ou bien
R(12) : groupe cycloalkyle-(C₃-C₈) ;
et où les substituants phényle, C₆H₅-alkyle-(C₁-C₂), naphtyle et biphénylyle, dans les groupes R(2) et R(3), ne sont pas substitués ou sont substitués par 1 à 3 substituants choisis parmi F, CI et les groupes CF₃, alkyle-(C₁-C₄), alcoxy-(C₁-C₄) et NR(10)R(11), R(10) et R(11) signifiant H ou un groupe alkyle-(C₁-C₄),
ainsi que leurs sels pharmaceutiquement acceptables.

3. Composé selon la revendication 1, caractérisé en ce que les groupes ont les significations suivantes :
R(1) : R(4)-SOₘ ou R(S)R(6)N-SO₂- ;
m: 0, 1 ou 2 ;
R(4) : groupe méthyle ou -CₙH₂ₘ-R(7) ;
n : 0 ou 1,
R(7) est un groupe phényle
non substitué ou substitué par 1 à 3 substituants choisis parmi Cl et les groupes CF₃, méthyle et méthoxy,
R(5) : H, groupe alkyle-(C₁-C₆), allyle, -CₙH₂ₙ-R(7),
n: 0, 1, 2 ou 3,
R(7) : groupe phényle
non substitué ou substitué par 1 à 3 substituants choisis parmi F, CI et les groupes CF₃, méthyle, méthoxy et NR(8)R(9) ;
R(8) et R(9) :
H ou groupe méthyle,
R(6) : H ou groupe méthyle,
ou bien
R(5) et R(6)
pouvant représenter ensemble 4 ou 5 groupes méthylène parmi lesquels un groupe CH₂ peut être remplacé par O, S, N-CH₃ ou un groupe N-benzyle,
R(2) : groupe alkyle-(C₅-C₈) linéaire ou ramifié, cycloalkyle-(C₃-C₈), cycloalkyl-(C₃-C₈)-alkyle-(C₁-C₂), phényle, C₆H₅-alkyle-(C₁-C₂), naphtyle, biphénylyle ou -C≡CR(12) ;
R(12) : groupe phényle
non substitué ou substitué par 1 à 3 substituants choisis parmi F, CI et les groupes CF₃, méthyle, méthoxy et NR(14)R(15) ;
R(14) et R(15) :
H ou groupe alkyle-(C₁-C₄) ;
ou bien
R(12) : groupe alkyle-(C₁-C₆)
non substitué ou substitué par 1 à 3 groupes OH ;
ou bien
R(12) : groupe cycloalkyle-(C₃-C₈) ;
R(3): atome d'hydrogène ou groupe phényle, cyclopentyle, C₆H₅-alkyle-(C₁-C₂) ou -C≡CR(12) ;
R(12) : groupe phényle
non substitué ou substitué par 1 à 3 substituants choisis parmi F, CI et les groupes CF₃, méthyle, méthoxy et NR(14)R(15) ;
R(14) et R(15) :
H ou groupe alkyle-(C₁-C₄),
ou bien
R(12) : groupe alkyle-(C₁-C₆)
non substitué ou substitué par 1 à 3 groupes OH,
ou bien
R(12) : groupe cycloalkyle-(C₃-C₈) ;
et où les substituants phényle, C₆H₅-alkyle-(C₁-C₂), naphtyle et biphénylyle, dans les groupes R(2) et R(3), ne sont pas substitués ou sont substitués par 1 à 3 substituants choisis parmi F, CI et les groupes CF₃, alkyle-(C₁-C₄), alcoxy-(C₁-C₄) et NR(10)R(11), R(10) et R(11) signifiant H ou un groupe alkyle-(C₁-C₄),
ainsi que leurs sels pharmaceutiquement acceptables.

4. Procédé pour la préparation des composés I, caractérisé en ce que l'on fait réagir des composés de formule Il 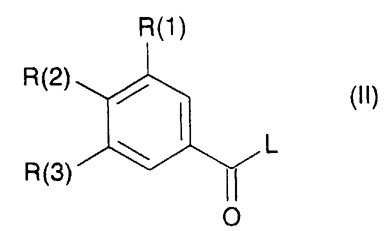 avec de la guanidine, les groupes R(1) à R(3) ayant les significations données et L signifiant un groupe partant, facilement substituable par un nucléophile.

5. Utilisation d'un composé I selon la revendication 1 pour la préparation d'un médicament pour le traitement d'arythmies.

6. Utilisation d'un composé I selon la revendication 1 pour la préparation d'un médicament pour le traitement ou la prophylaxie de l'infarctus du myocarde.

7. Utilisation d'un composé I selon la revendication 1 pour la préparation d'un médicament pour le traitement de l'angine de poitrine.

8. Utilisation d'un composé I selon la revendication 1 pour la préparation d'un médicament pour le traitement des états ischémiques du coeur.

9. Utilisation d'un composé I selon la revendication 1 pour la préparation d'un médicament pour le traitement de maladies dont une cause primaire ou secondaire est représentée par la prolifération des fibroblastes, et ainsi son utilisation comme antiathérosclérotique, comme agent contre les complications tardives du diabète, les maladies cancéreuses, les maladies fibrotiques telles que les fibromes des poumons, du foie ou des reins.

10. Utilisation d'un composé I selon la revendication 1 pour la préparation d'un outil scientifique pour l'inhibition de l'échangeur-Na⁺/H⁺ pour le diagnostic de l'hypertonie et de maladies prolifératives.
